(19) Europäisches Patentamt
European Patent Office
Office européen des brevets

(11) Veröffentlichungsnummer : **0 080 137**
**B1**

(12) **EUROPÄISCHE PATENTSCHRIFT**

(45) Veröffentlichungstag der Patentschrift : **02.01.86**

(51) Int. Cl.⁴ : **C 07 C135/02**, C 11 D 1/90

(21) Anmeldenummer : **82110487.4**

(22) Anmeldetag : **13.11.82**

(54) **Bis-Betain-Aminoxide, Verfahren zu deren Herstellung und diese enthaltende Reinigungsmittel.**

(30) Priorität : **19.11.81 DE 3145734**

(43) Veröffentlichungstag der Anmeldung : **01.06.83 Patentblatt 83/22**

(45) Bekanntmachung des Hinweises auf die Patenterteilung : **02.01.86 Patentblatt 86/01**

(84) Benannte Vertragsstaaten : **AT BE CH DE FR GB IT LI NL SE**

(56) Entgegenhaltungen :
**EP-A- 0 060 710**
**DE-A- 2 063 422**
**US-A- 3 265 719**

(73) Patentinhaber : **HOECHST AKTIENGESELLSCHAFT**
**Postfach 80 03 20**
**D-6230 Frankfurt am Main 80 (DE)**

(72) Erfinder : **Blaschke, Günter, Dr.**
**Bajuwarenstrasse 36**
**D-8261 Winhöring (DE)**
Erfinder : **Reng, Alwin**
**Im Schulzehnten 22**
**D-6233 Kelkheim (Taunus) (DE)**
Erfinder : **Quack, Jochen Meinhard, Dr.**
**Wilhelm-Reuter-Strasse 16**
**D-6239 Eppstein/Taunus (DE)**

**EP 0 080 137 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die menschliche Haut und Haare werden sowohl durch körperfremde als auch körpereigene Verunreinigungen belastet. Hauptsächlisch handelt es sich hierbei um Pigmente, wie Eisenoxide, Siliciumdioxid, Ruß und Öle sowie Fette (Sebum), deren Oxidations- und Abbauprodukte und Mikroorganismen. Zur Entfernung dieser Verunreinigungen werden im allgemeinen tensidhaltige Lösungen oder Dispersionen benutzt. Gebräuchlich sind Tenside mit anionischer, nicht-ionischer, amphoterer oder eventuell kationischer Struktur. Am häufigsten werden Seifen, Alkylsulfate, Alkylethersulfate, Alkylbenzolsulfonate oder Alkansulfonate allein oder in Kombination zur Haut- und Haarreinigung verwendet. Die meisten dieser Tenside sind durch eine sehr ausgeprägte Reinigungswirkung charakterisiert. Bei der Durchführung des Waschprozesses mit den vorgenannten Tensiden kommt es von seiten des Verbrauchers häufig zu einer Überdosierung ; die Folge ist eine zu starke Entfettung der Haare und Hautoberfläche, die sich in einer schlechten Frisierbarkeit der Haare und einem trockenen « spannenden » und unangenehmen Haut- und Kopfhautgefühl äußert. Gleichzeitig wird durch die Adsorption der anionischen Tenside ein unangenehmes « klebriges » Hautgefühl nach der Anwendung beobachtet. Außerdem zeigt die Haut nach langzeitiger Anwendung solcher Tenside oft eine rauhe und rissige Oberfläche. Es wurde schon versucht, durch Zusatz von kationischen Tensiden diese Nachteile zu umgehen, wobei es jedoch in den Formulierungen durch die Bildung von Elektroneutralsalzen zwischen anionischen und kationischen Tensiden oft zu Trübungen und Ausfällungen kommt. Auch der Zusatz von polymeren, kationischen Substanzen verursacht oft Schwierigkeiten durch Reduzierung der Schaumbildung und zu hohe Adsorption auf den Haaren, die zu einem unangenehmen Akkumulationseffekt führt. Die Folge ist ein « fettiger » Griff der gewaschenen Kopfhaare.

Auch amphotere Tenside mit einer Betain-Gruppe im Molekül sind versuchsweise allein oder in Kombination mit anionischen Tensiden benutzt worden. Aus der DE-AS 12 49 433 ist beispielsweise der Einsatz von Alkyl-Betainen in Reinigungsmitteln bekannt, während Amidoalkyl-Betaine der Formel $R^1 CONH \cdot (CH_2)_x \cdot N^+R^2R^3 \cdot (CH_2)_y \cdot COO^-$, worin $R^1$ der Alkylrest einer Fettsäure ist, in der DE-AS 11 72 802 als hautverträgliche Badezusätze, in der DE-AS 10 62 392 als keimtötende, die Augen nicht reizende Haarwaschmittel empfohlen worden sind. Jedoch ist die tensidische Wirkung dieser Mono-Betaine nicht ausreichend. Außerdem besitzen beispielsweise die Alkyl-Betaine eine schlechte Augenschleimhaut-Verträglichkeit und auch diejenige der Amidoalkyl-Betaine ist noch nicht optimal.

Zur Lösung aller dieser Probleme besteht ein Bedürfnis nach sogenannten milden Tensiden mit schwach ausgeprägtem Reinigungseffekt, die auch dem zeitgemäßen Bedarf nach kürzeren Waschintervallen entgegenkommen und auch beispielsweise eine erhöhte Waschfrequenz bei der Haarreinigung oder beim Duschen ermöglichen. Gleichzeitig können durch die milde Reinigungswirkung die in der Praxis üblichen Überkonzentrationen der Tensidlösungen kompensiert werden.

Erfindungsgemäß werden dafür Bis-Betain-Aminoxide der allgemeinen Formel

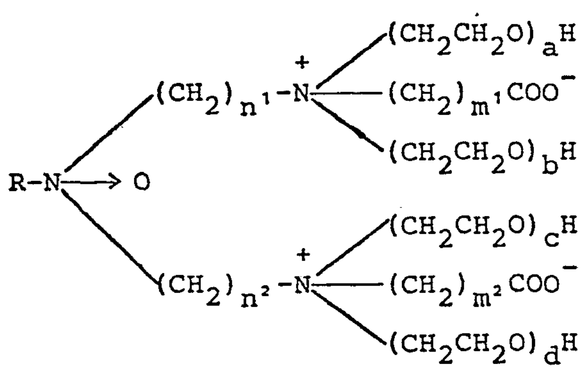

(I)

zur Verfügung gestellt, worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt, sowie a, b, c und d, gleich oder verschieden, jeweils eine Zahl von 1 bis 5 ist, wobei die Summe $a + b + c + d$ höchstens 10 betragen soll.

In diesen erfindungsgemäßen Bis-Betain-Aminoxiden der Formel I besitzt der Rest R 8 bis 22 C-Atome, er kann gesättigt oder ungesättigt mit 1 bis 3 olefinischen Doppelbindungen und er kann geradkettig oder verzweigt sein. Diese Alkyl- oder Alkenylreste, die dem primären Ausgangsamin bei der Herstellung der erfindungsgemäßen Bis-Betain-Aminoxide entstammen, sind häufig Gemische oder Kettenschnitte, bevorzugt mit der Kettenverteilung der Reste von natürlichen Fettsäuren, wie insbesondere der Kokos-, Talg- oder Palmkernfettsäure, aus denen diese Ausgangsamine über den Weg der Nitrilhydrierung oder der Ammonolyse der entsprechenden Alkohole gewonnen werden. Die zur Herstellung der primären Amine mittels Ammonolyse verwendeten Alkohole können neben Fettalkoholen auch solche mit gerader oder verzweigter Kette aus dem Ziegler-Prozeß (Ethylen-Aufbaualkohole) oder aus der Oxosynthese sein.

Zur Herstellung der erfindungsgemäßen Verbindungen wird zunächst ein solches primäres Amin der Formel $RNH_2$ (II), wobei R die vorgenannte Bedeutung hat, mit 2 mol mindestens eines reaktionsfähigen Nitrils von 2 bis 3 C-Atomen (einschließlich der CN-Gruppe) zu einer Verbindung der allgemeinen Formel

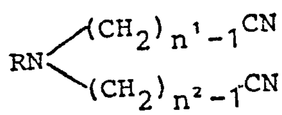

(III)

in einer Dicyanalkylierungsreaktion umgesetzt. Diese Reaktion ist bekannt, beispielsweise aus der US-PS 3 028 415. Sie kann sowohl mit saurer als auch mit basischer Katalyse, mit Hilfe von Lösungsmitteln, wie Wasser oder auch niederkettigen Alkoholen, drucklos oder unter erhöhtem Druck, kontinuierlich oder diskontinuierlich durchgeführt werden. Als saure Katalysatoren werden Essigsäure, Phosphorsäure, Salzsäure und andere Mineralsäuren genannt (US-PS 3 615 797, US-PS 3 028 415, DE-OS 1 941 913), als basische Katalysatoren sind empfohlen worden Natrium- oder Kaliumhydroxid, Alkalialkoholate, Trimethylbenzylammoniumhydroxid und Morpholin (Kirk-Othmer, Encyclopedia of Chemical Technology, 1965, Band 6, Seite 634 ff. ; H. A. Bruson « Cyanoethylation », Organic Reactions 5, 1949, Seite 79 ff., Verlag John Wiley and Sons, New York). Als Co-Katalysatoren oder auch als Lösungsvermittler werden Wasser oder niedere Alkohole, wie Methanol, Ethanol, Isopropanol oder Gemische derselben in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines Inertgases, und bei Temperaturen von 60 bis 150 °C durchgeführt. Das Cyanalkylierungsmittel, vorzugsweise Acrylnitril oder Chloracetonitril, wird stöchiometrisch oder in einem bis zu vierfachen Überschuß angewandt.

Anschließend wird das so gewonnene Dicyanalkylierungsprodukt (III) in Gegenwart von Wasserstoff zu einer Verbindung der Formel

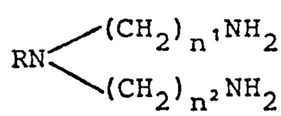

(IV)

reduziert und anschließend mit Ethylenoxid zu einer Verbindung der Formel

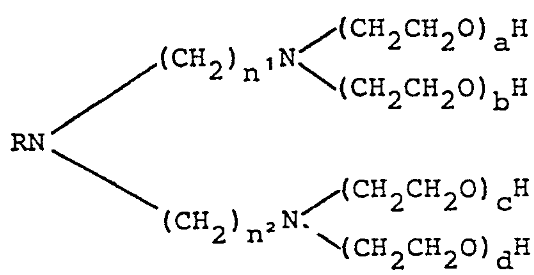

(V)

kondensiert. Beide Reaktionen sind für die in Rede stehenden Verbindungen ebenfalls bekannt (vgl. die oben bereits genannte US-PS 3 615 797). Die Reduktion wird mit Raney-Nickel oder Raney-Kobalt oder aber mit Nickel- oder aber mit Nickel- oder Kobalt-Trägerkatalysatoren durchgeführt, und zwar unter Einsatz von 1 bis 10 Gew.-% Katalysator, bevorzugt 1 bis 5 Gew.-%, und bei Drücken von 50 bis 200 bar Wasserstoff und Temperaturen von 60 bis 150 °C ; die Reaktionszeit beträgt dabei etwa 1 bis 5 Stunden.

Die Oxethylierungsreaktion wird in Druckgefäßen durchgeführt, und zwar bei erhöhter Temperatur im Bereich von 110 bis 170 °C und erhöhtem Druck von 1 bis 5 bar. Ein Katalysator ist nicht erforderlich, wenn vorzugsweise nur eine Ethylenoxid-Einheit pro Kette angelagert werden soll. Wird ein Katalysator eingesetzt, so ergeben sich vorzugsweise Ethylenoxidketten, die mehr als eine Einheit enthalten. Es werden 4 bis 10 mol Ethylenoxid pro 1 mol der Verbindung IV in der Reaktion eingesetzt, vorzugsweise 4 bis 5 mol. Das Ethylenoxid kann dabei mit einem Inertgas verdünnt werden.

Danach wird das so erhaltene Ethylenoxid-Anlagerungsprodukt in an sich bekannter Weise zu dem Bis-Betain der Formel

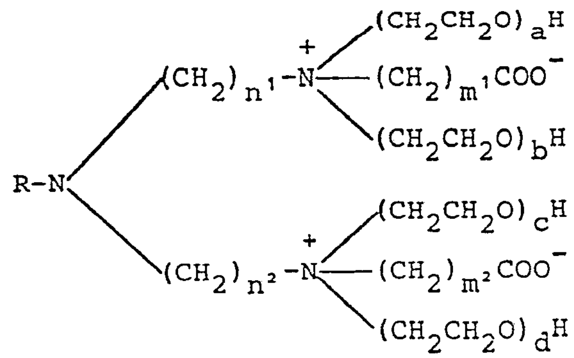

(VII)

umgesetzt, und zwar in wäßriger Lösung mit mindestens einem Alkalisalz einer ω-Halogencarbonsäure der Formel $X(CH_2)_{m^1(m^2)}COOH$ (VI) ; vorzugsweise seien genannt die Alkalisalze und insbesondere die Natriumsalze der Chloressigsäure, Chlorpropionsäure, Bromessigsäure und der Chlor-n-buttersäure. Gegebenenfalls können Halogencarbonsäure und Alkalihydroxid getrennt zugegeben werden, wobei sich das Salz in situ bildet. Diese Reaktion wird bei 80 bis 100 °C mit einem 5 bis 10 %igen Überschuß an Halogencarbonsäure durchgeführt.

Die noch verbleibende freie Aminogruppe in dem so erhaltenen Bis-Betain der Formel (VII) wird anschließend mit 70 %igem Wasserstoffperoxid, das in 5 bis 10 %igem molarem Überschuß angewandt wird, bei einer Temperatur von 70 bis 90 °C zu Bis-Betain-Aminoxid der Formel I oxidiert.

Vorteilhafterweise stellt man durch geeignete Wahl des Wassergehaltes bei der letzten Reaktionsstufe die erfindungsgemäßen Bis-Betain-Aminoxide der Formel I als 30 bis 40 gew.-%ige wäßrige Einstellungen her.

Die erfindungsgemäßen Bis-Betain-Aminoxide, wie sie in der obengenannten Formel I definiert sind, sind mild wirkende Tenside mit schwach entfettender Wirkung und daher hervorragend geeignet zum Einsatz in kosmetischen Reinigungsmitteln, das heißt Körperreinigungsmitteln wie Schaumbädern, Duschbädern, Fuß- und Handwaschmitteln oder Intimwaschmitteln sowie ferner in Haarwaschmitteln. Beim Einsatz in Körperreinigungsmitteln wird eine deutliche Verbesserung des Hautgefühls nach der Anwendung erzielt, beim Einsatz in Shampoos wird eine Verbesserung der Kämmbarkeit sowohl der trockenen als auch der nassen Haare erreicht bei gleichzeitig weichmachender Wirkung, die sich in einem angenehmen Haargriff bemerkbar macht.

Die erfindungsgemäßen Bis-Betain-Aminoxide können in solchen flüssigen, pulverförmigen oder auch aerosolförmigen kosmetischen Reinigungsmitteln, insbesondere in Haarwaschmitteln, sowohl allein als auch in Kombination mit den üblicherweise in solchen Mitteln eingesetzten anionischen, kationischen, nicht-ionischen und amphoteren Tensiden verwendet werden. Dafür geeignete anionische Tenside sind beispielsweise Seife, Fettalkoholsulfate, Alkylethersulfate, Fettsäurekondensationsprodukte wie Tauride, Methyltauride, Sarkoside, ferner α-Olefinsulfonate, Hydroxyalkansulfonate, sekundäre Alkansulfonate, Amidethersulfate oder Alkylbenzolsulfonate. Als nicht-ionische Tenside können beispielsweise Polyglykolmonoalkylether und -monoester, Aminoxide und Ethylenoxid-Propylenoxid-Kondensationsprodukte verwendet werden. Daneben ist auch die Kombination mit anderen amphoteren Tensiden wie Alkyl-Betainen, Alkylamido-Betainen, Imidazolinderivaten oder Sulfo-Betainen möglich. Schließlich können die erfindungsgemäßen Bis-Betain-Aminoxide auch in Abmischung mit kationischen Tensiden wie Cetyltrimethylammoniumchlorid, Cetyltrimethylammoniumbromid, Pentaoxyethylstearylammoniumchlorid, quaternierten Etheraminen oder polymeren quartären Ammoniumverbindungen eingesetzt werden. Weitere Zusätze, die auf sonst übliche Weise in kosmetischen Reinigungsmitteln verwendet werden, können mit den Bis-Betain-Aminoxiden kombiniert werden. Dies sind beispielsweise viskositätserhöhende oder -erniedrigende Verbindungen wie Celluloseether, Elektrolyte, wie beispielsweise Natriumchlorid oder Ammoniumchlorid, Fettsäurepolyglykolester, Alkanolamide, Magnesium-Aluminium-Silicate, Polyglykole, Glycerin und Ethanol. Ferner kommen als solche Zusätze in Frage Parfümöle und spezielle Riechstoffe, Antiseptika, schuppenentfernende oder pilztötende Mittel, Überfettungsmittel, Konservierungsmittel, Farbstoffe und Perlglanz gebende Substanzen. Bei der Verarbeitung zu pulverförmigen Präparaten können weiterhin üblicherweise benutzte Füll- und Trägersubstanzen wie hochdisperse, amorphe Kieselsäure, Natriumsulfat, Magnesium-Aluminium-Silicat, Stärkederivate und dergleichen verwendet werden. Schließlich können auch im Falle von aerosolförmigen Präparaten übliche Treibgase beigemischt werden. Die Regulierung des gewünschten pH-Wertes kann mit anorganischen oder organischen Säuren oder Alkalien vorgenommen werden.

Weiterhin sind die erfindungsgemäßen Bis-Betain-Aminoxide auch zur Formulierung von technischen Reinigungsmitteln, also Schaumreinigern für textile Flächen wie Teppichreiniger, oder insbesondere Reinigungsmittel für harte Oberflächen, wie beispielsweise Geschirr- und Flaschenspülmittel, Fußbodenreiniger, Sanitärreiniger oder sogenannte Allzweckreinigungsmittel, geeignet. Schließlich eignen sich die erfindungsgemäßen Bis-Betain-Aminoxide als Textilwaschmittel. Auch bei diesen Anwendungsmöglichkeiten können die obengenannten anionischen, kationischen, nicht-ionischen oder

amphoteren Tenside beigemischt werden. In technischen Reinigungsmitteln können dabei als übliche Hilfsmittel Chelatbildner und gegebenenfalls auch Kunststoffdispersionen zugesetzt werden. Ferner sind übliche Zusätze hier Bleichmittel, Chlorabspalter oder andere Desinfektionsmittel. Zur Verbesserung der Abrasionswirkung eignen sich Kreide, hochdisperse amorphe Kieselsäure, Phosphate und Kunststoffe. Zur Verbesserung der Fett- und Schmutzlöseeigenschaften können auch Lösungsmittel wie Testbenzin oder Isopropylalkohol oder andere reinigungsverstärkende Mittel zugefügt werden. Schließlich enthalten Waschmittel die üblichen Gerüstsubstanzen.

Ein besonderer anwendungstechnischer Vorteil beim Einsatz der Bis-Betain-Aminoxide in technischen und kosmetischen Reinigungsmitteln ist ihre physikalische und insbesondere auch ihre chemische Stabilität, also beispielsweise gegenüber auch in hoher Konzentration anwesender Elektrolyte, gegenüber Bleichmitteln, wie sie in Sanitärreinigern anwesend sind, gegenüber starken Alkalien, wie sie in Flaschen- oder Backofenreiniger eingearbeitet werden, oder gegenüber Oxidationsmitteln, wie sie zum Beispiel in Dauerwellenpräparaten anwesend sind. Beispielsweise ist es auch möglich, aus den erfindungsgemäßen Bis-Betain-Aminoxiden lagerstabile Shampoos mit saurem pH-Wert herzustellen, ohne daß — im Gegensatz zu den üblicherweise verwendeten anionischen Alkylsulfaten oder Alkylethersulfaten — Zersetzung durch Hydrolyse eintritt.

Der Gehalt an den erfindungsgemäßen Bis-Betain-Aminoxiden in solchen Formulierungen beträgt üblicherweise 0,5 bis 40 Gew.-%.

Die folgenden Beispiele sollen die Erfindung näher erläutern :

## Herstellungsbeispiele

### Beispiel 1

In einen 2-l-Vierhalskolben mit Rückfluß, Thermometer, Rührer und Dosiergefäß werden 670 g Cocosfettamin (Zusammensetzung in Mol-% bezüglich der Reste R : $C_8$ 6 %, $C_{10}$ 6 %, $C_{12}$ 54 %, $C_{14}$ 18 %, $C_{16}$ 8 %, $C_{18}$ 8 %), 68 g Wasser, 34 g Methanol und 14 g konzentrierte Essigsäure auf 60 °C erhitzt. Innerhalb einer Stunde tropft man 373 g Acrylnitril zu und rührt weitere 24 bis 36 h bei 75 °C unter Rückfluß. Anschließend wird mit 13 g NaOH und 120 g Wasser neutralisiert, das Waschwasser separiert und das Produkt im Vakuum von Restwasser und Lösungsmittel befreit. Man erhält 1 000 g Cocosfettamino-di-propionitril (Ausbeute 95,9 %).

Ein 5-l-Autoklav wird mit 2 020 g Cocosfettamino-di-propionitril, 3 g Kobalt-Trägerkontakt (Träger : Kieselgur) und 300 ml flüssigen Ammoniaks beschickt. Die Hydrierung erfolgt bei 150 bis 180 bar $H_2$ und 110 bis 140 °C innerhalb von 3 Stunden. Nach Abfiltrieren des Katalysators erhält man 2 010 g Produkt, das 85 bis 95 % Bis(3-aminopropvl)-cocosfettamin enthält.

In einem 2-l-Druckgefäß mit Thermometer, Rührer und Ethylenoxid-Einlaß und -Auslaß werden 954 g Bis(3-aminopropyl)-cocosfettamin auf 130 °C unter Rühren aufgeheizt. 667 g Ethylenoxid werden bei einem Druck von 1 bis 3 bar aufgegeben. Die Gewichtszunahme nach 3 Stunden Reaktionszeit entspricht einem Kondensationsprodukt des Triamins mit 4 bis 5 mol Ethylenoxid. Man erhält 1 605 g dieses Oxethylats (99 %).

237 g Bis(3-aminopropyl)-cocosfettaminoxethylat und 609 g Wasser werden in einem 2-l-Reaktionsgefäß vorgelegt und unter Rühren auf 90 °C erwärmt. Bei dieser Temperatur werden innerhalb 1 h 110 g Natriumchloracetat zugegeben, danach wird noch 12 h bei 95 °C nachgerührt. Man erhält das erfindungsgemäße Bis-Betain-Aminoxid durch anschließende Zugabe von 43 g 70 %igem Wasserstoffperoxid und einer Nachreaktionszeit von 8 h bei 80 °C als 30 %ige wäßrige Lösung.

### Beispiel 2

670 g Laurylamin (Anteil $C_{12}$ 73 Mol-%, Anteil $C_{14}$ 23 Mol-%) werden 68 g Wasser, 34 g Methanol und 14 g konzentrierter Essigsäure in der bereits in Beispiel 1 beschriebenen Weise mit 373 g Acrylnitril umgesetzt und dann hydriert.

Nach der Hydrierung erhält man 2 020 g Bis(3-aminopropyl)-laurylamin.954 g dieses Triamins werden mit 640 g Ethylenoxid kondensiert. Man erhält 1 590 g an Bis(3-aminopropyl)-laurylaminoxethylat (99 %).

257 g dieses Oxethylats und 655 g Wasser werden mit 116,5 g Natriumchloracetat und anschließend mit 48,6 g 70 %igem $H_2O_2$ zur Reaktion gebracht. Man erhält das Bis-Betain-Aminoxid in 1 070 g einer 30 %igen wäßrigen Lösung.

### Beispiel 3

844 g Myristylamin werden in 68 g Wasser, 34 g Methanol und 14 g konzentrierter Essigsäure mit 373 g Acrylnitril bei 75 °C umgesetzt. Man erhält 1 164 g Myristylamino-di-propionitril. 2 052 g des Dipropionitrils werden wie in Beispiel 1 mit Kobalt-Katalysator hydriert.

Man erhält 2 045 g Bis(3-aminopropyl)-myristylamin. 1 095 g dieses Triamins werden mit 647 g Ethylenoxid kondensiert. Man erhält 1 725 g an Bis(3-aminopropyl)-myristylaminoxethylat (99 %). 290 g

dieses Oxethylats und 733 g Wasser werden mit 116,5 g Natriumchloracetat und anschließend mit 48,6 g 70 gew.-%igem $H_2O_2$ zur Reaktion gebracht. Man erhält das Bis-Betain-Aminoxid in 1 180 g einer 30 %igen wäßrigen Lösung.

## Beispiel 4

1 056,0 g Octylamin werden in 106 g Wasser, 53 g Methanol und 21,1 g konzentrierter Essigsäure mit 849,6 g Acrylnitril bei 75 °C umgesetzt. Man erhält 1 810 g Octylamino-di-propionitril. 1 980 g des Dipropionitrils werden wie in Beispiel 1 mit Kobalt-Katalysator hydriert. Man erhält 1 970 g Bis(3-aminopropyl)-octylamin. 998 g dieses Triamins werden mit 845 g Ethylenoxid kondensiert. Man erhält 1 840 g an Bis(3-aminopropyl)-octylaminoxethylat (99 %). 230 g dieses Oxethylats und 593 g Wasser werden mit 116,5 g Natriumchloracetat und anschließend mit 48,6 g 70 Gew.-%igem $H_2O_2$ zur Reaktion gebracht. Man erhält das Bis-Betain-Aminoxid in 960 g einer 30 %igen wäßrigen Lösung.

## Beispiel 5

929 g Talgfettamin werden in 68 g Wasser, 34 g Methanol und 14 g konzentrierter Essigsäure mit 373 g Acrylnitril bei 75 °C umgesetzt. Man erhält 1 237 g Talgfettamino-di-propionitril, das gemäß Beispiel 1 zum entsprechenden Amin hydriert wird. Man erhält 1 230 g Bis(3-aminopropyl)-talgfettamin.

Die Oxethylierungsreaktion wird in zwei Stufen durchgeführt. Zunächst werden 1 154 g Bis(3-aminopropyl)-talgfettamin nach der Methode von Beispiel 1 mit 647 g (4,7 mol) Ethylenoxid umgesetzt. Unter Einsatz von üblichen 0,2 Gew.-%, bezogen auf das Amin, an wäßriger Natronlauge (50 %ig) wird anschließend mit weiteren 5,3 mol Ethylenoxid umgesetzt, so daß die gesamte Gewichtszunahme einem Kondensationsprodukt des Triamins mit 10 mol Ethylenoxid entspricht. 300 g dieses Oxethylats und 756 g Wasser werden mit 116,5 g Natriumchloracetat und anschließend mit 48,6 g 70 gew.-%igem $H_2O_2$ umgesetzt. Man erhält das Bis-Betain-Aminoxid in 1 216 g einer 30 %igen wäßrigen Lösung.

Die analytischen Daten der erfindungsgemäßen Bis-Betain-Aminoxide und deren Vorprodukte sind in Tabelle I zusammengefaßt.

(Siehe Tabelle 1 Seite 7 f.)

Tabelle I

| Beispiel/ (Alkylrest) | Alkylamino-di-propionitril | | Bis(3-aminopropyl)-alkyl-amin | | | | O x e t h y l a t | | | Bis-Betain-Aminoxid | | |
|---|---|---|---|---|---|---|---|---|---|---|---|---|
| | AZ | tert. N (%) | AZ | prim. N (%) | sek. N (%) | tert. N (%) | AZ | tert. N (%) | Σ a + b + c + d Äquiv. EO* pro mol | Gesamt-Chlor Gew.-% | ionogenes Chlor Gew.-% | Gehalt Gew.-% |
| 1 (Cocos) | 34,3 | 94 | 97,3 | 66,2 | 3,3 | 30,5 | 57,5 | >98 | 4,9 | 3,5 | 3,4 | 29,4 |
| 2 (Lauryl) | 34,4 | 95 | 97,5 | 65,9 | 2,9 | 31,2 | 58,3 | >98 | 4,7 | 3,5 | 3,4 | 29,5 |
| 3 (Myristyl) | 29,2 | 94 | 82,2 | 65,5 | 3,5 | 31,0 | 51,7 | >98 | 4,9 | 3,4 | 3,3 | 29,1 |
| 4 (Octyl) | 42,7 | 94 | 120,2 | 65,1 | 3,0 | 31,9 | 65,1 | >98 | 4,8 | 3,6 | 3,5 | 29,4 |
| 5 (Talg-fett) | 27,4 | 94 | 78,0 | 66,1 | 2,6 | 31,3 | 50,0 | >98 | 4,9 | 3,0 | 3,0 | 29,9 |

* EO = Ethylenoxid

Die obengenannten Daten werden wie folgt bestimmt :

Alkylamino-di-propionitril :

Die Bestimmung der Aminzahl (AZ) und des Gehaltes an tertiärem Stickstoff erfolgt durch Titration mit 0,1 N $HClO_4$ in Eisessig bzw. Essigsäureanhydrid. Die Aminzahl errechnet sich aus

$$AZ = \frac{ml\ 0{,}1\ N\ HClO_4}{Einwaage\ in\ g}$$

Bis(3-aminopropyl)-alkylamin :

Die Bestimmung der Aminzahl und der Aminverteilung erfolgt durch Titration mit 0,2 N-isopropanolischer HCl in wasserfreiem Medium. Die Aminverteilung wird durch Blockierung des basischen Aminstickstoffs mit Salicylaldehyd (primäres N) bzw. Phenylisothiocyanat (primäres und sekundäres N) durchgeführt.

Oxethylat :

Die Bestimmung der Aminzahl und des Gehaltes an tertiärem Stickstoff erfolgt durch Titration mit 0,1 N $HClO_4$ in Eisessig bzw. Essigsäureanhydrid.

Die aufgenommenen Mole Ethylenoxid errechnen sich aus den Aminzahlen bzw. aus der Massenzunahme gegenüber der Vorstufe.

Bis-Betain-Aminoxid :

Die Bestimmung des Gehaltes an Gesamtchlor erfolgt nach Parr-Aufschluß mit $Na_2O_2$, die Bestimmung des ionogenen Chlors erfolgt durch Titration nach Volhard. Der Gehalt an Aminoxid wird durch Redoxtitration mit Ti(III)-chlorid/$NH_4Fe(SO_4)_2 \cdot 6H_2O$ bestimmt.

Die nachfolgenden Anwendungsbeispiele veranschaulichen die Einsatzmöglichkeiten der Bis-Betain-Aminoxide zur Herstellung von milden Reinigungsmitteln.

Die Mengen und Prozentangaben in den Beispielen beziehen sich auf das Gewicht.

Beispiel 1 A

| | |
|---|---|
| Shampoo für jeden Tag | |
| Bis-Betain-Aminoxid der Formel I, hergestellt nach Beispiel 1 | 15,0 % |
| Hydroxyethylcelluloseether | 1,3 % |
| Parfümöl | 0,2 % |
| Wasser, Konservierungsmittel, Farbstoff | ad 100,0 % |

Beispiel 2 A

| | |
|---|---|
| Shampoo für trockene Haare | |
| Bis-Betain-Aminoxid der Formel I, hergestellt nach Beispiel 2 | 10,0 % |
| Lauryldiglykolethersulfat-Natriumsalz | 5,0 % |
| Polyethylenglykol-6 000-distearat | 5,0 % |
| Wasser, Konservierungsmittel, Farbstoff | ad 100,0 % |

Beispiel 3 A

| | |
|---|---|
| Duschbad | |
| Bis-Betain-Aminoxid der Formel I, hergestellt nach Beispiel 1 | 8,0 % |
| Cocostriglykolethersulfosuccinat-Dinatriumsalz | 6,0 % |
| Cocosfettsäure-monoethanolamid | 1,0 % |
| Hydroxyethylcelluloseether | 1,2 % |
| Wasser, Konservierungsmittel, Parfümöl | ad 100,0 % |

Beispiel 4 A

| | |
|---|---|
| Intim-Waschmittel | |
| Bis-Betain-Aminoxid der Formel I, hergestellt nach Beispiel 1 | 10,0 % |
| Cocosethylcycloimidino-1-hydroxy-3-ethylnatriumalkoholat-2-methylnatriumcarboxylat | 5,0 % |
| Citronensäure | 0,2 % |
| Bakteriostatikum, Parfümol, Wasser | ad 100,0 % |

Beispiel 5 A

| | |
|---|---|
| Handwaschmittel | |
| Bis-Betain-Aminoxid der Formel I, hergestellt nach Beispiel 1 | 10,0 % |
| sekundäres Alkansulfonat-Natriumsalz (Alkanrest $C_{13}$-$C_{17}$) | 5,0 % |
| Cocosfettsäurediethanolamid | 2,0 % |
| Wasser, Konsistenzgeber, Parfümöl | ad 100,0 % |

Beispiel 6 A

| | |
|---|---|
| Saures Shampoo | |
| Bis-Betain-Aminoxid der Formel I, hergestellt nach Beispiel 2 | 7,0 % |
| Acylaminoglykolethersulfat-Triethanolaminsalz (Acyl = Capryl-bis Stearinsäurerest) | 5,0 % |
| Lauroylsarkosid-Natriumsalz | 2,0 % |
| Citronensäure | 0,45 % |
| Konsistenzgeber, Farbstoff, Konservierungsmittel, Wasser, Parfümöl | ad 100,0 % |

Beispiel 7 A

| | |
|---|---|
| Schaumbad | |
| Bis-Betain-Aminoxid der Formel I, hergestellt nach Beispiel 2 | 20,0 % |
| Laurylsulfat-Triethanolaminsalz | 5,0 % |
| Laurylalkohol, kondensiert mit 10 mol Ethylenoxid | 3,0 % |
| Ölsäureethanolamid | 1,0 % |
| Wasser | ad 100,0 % |

Beispiel 8 A

| | |
|---|---|
| Schaumbad | |
| Bis-Betain-Aminoxid der Formel I, hergestellt nach Beispiel 1 | 35,0 % |
| Cocosfettsäurediethanolamid | 2,0 % |
| Parfümöl | 1,0 % |
| Wasser | ad 100,0 % |

Beispiel 9 A

| | |
|---|---|
| Babyshampoo | |
| Bis-Betain-Aminoxid der Formel I, hergestellt nach Beispiel 2 | 12,0 % |
| Capryl-/Caprinsäure-triglycerid | 3,0 % |
| Kamillenextrakt | 0,1 % |
| Wasser, Konservierungsmittel | ad 100,0 % |

Beispiel 10 A

| | |
|---|---|
| Technisches Reinigungsmittel | |
| Bis-Betain-Aminoxid der Formel I, hergestellt nach Beispiel 1 | 20,0 % |
| Isopropylalkohol | 5,0 % |
| Harnstoff | 5,0 % |
| Wasser, Parfümöl | ad 100,0 % |

Um die schwach ausgeprägte Reinigungswirkung zu bestimmen, wurden die erfindungsgemäßen Tenside in nachstehenden in-vitro-Prüfungen untersucht :

1. Pigmentwaschvermögen im Launder-O-Meter

Bei diesem Test wurde als Apparatur ein Launder-O-Meter (Hersteller : Firma Atlas Electric Divices, Chicago 13, USA) benutzt. Hierbei wurde ein mit Mineralöl und Eisenoxiden angeschmutztes Wollgewebe der Wäschereiforschungsanstalt Krefeld unter den nachstehenden definierten Bedingungen gewaschen :
Tensid-Konzentration : 0,5 und 1 %
Wasser : 15° deutscher Härte
Waschtemperatur : + 40 °C
Waschzeit : 10 min
Mechanik : 10 Stahlkugeln
Nach Durchführung der Waschversuche wurden die Wollstücke auf einem Kalander getrocknet und die Remission mit einem Elrepho-Weißgrad-Meßgerät ermittelt.

$$\% \text{ Aufhellung} = \frac{(\text{Remissionswert-Blindwert}) \times 100}{\text{max. Remission-Blindwert}}$$

In der Tabelle II sind die entsprechenden Zahlenwerte aufgelistet.

Es zeigt sich, daß die erfindungsgemäßen Bis-Betain-Aminoxide eine wesentlich mildere Waschwirkung als die Standardtenside aufweisen.

2. Solubilisiertest mit Isopropylmyristat

Für die Reinigung von verschmutzten Oberflächen spielt neben der Dispergierung der Teilchen in der wäßrigen Phase auch die Solubilisierung bzw. Emulgierung von ölförmigen Verunreinigungen eine Rolle. Um das Solubilisiervermögen von Tensiden zu charakterisieren, wurde eine rot angefärbte Isopropylmyristatlösung in eine 10 %ige tensidhaltige wäßrige Lösung eingerührt ; nach 60 minütigem Rühren bei 300 Upm folgte eine 24 stündige Lagerung der Lösung bzw. Emulsion bei + 20 °C im Scheidetrichter. Anschließend wird von der unteren klaren Phase kolorimetrisch die Menge an solubilisiertem Isopropylmyristat bestimmt.

Die Tabelle III zeigt das geringe Solubilisiervermögen der erfindungsgemäßen Bis-Betain-Aminoxide im Vergleich zu den handelsüblichen stark reinigenden Substanzen.

Tabelle II

| Produkt (1 % Aktivsubstanz) | % Aufhellung |
|---|---|
| Alkylbenzolsulfonat-Natriumsalz | 79,5 |
| Lauryldimethylaminoxid | 62,4 |
| Laurylsulfat-Natriumsalz | 46,1 |
| Cocosamidobetain | 74,1 |
| Bis-Betain-Aminoxid von Beispiel 2 (erfindungsgemäß) | 16,2 |

Tabelle III

| Produkt (10 % Aktivsubstanz in Isopropylmyristat) | g solubilisiertes Isopropylmyristat |
|---|---|
| Alkylbenzolsulfonat-Natriumsalz | 2,450 |
| Lauryldimethylaminoxid | 3,263 |
| Laurylsulfat-Natriumsalz | 2,831 |
| Cocosamidobetain | 1,266 |
| Bis-Betain-Aminoxid von Beispiel 2 (erfindungsgemäß) | 0,081 |

**Patentansprüche** (für die Vertragsstaaten : BE, CH, DE, FR, GB, IT, LI, NL, SE)

1. Bis-Betain-Aminoxide der allgemeinen Formel

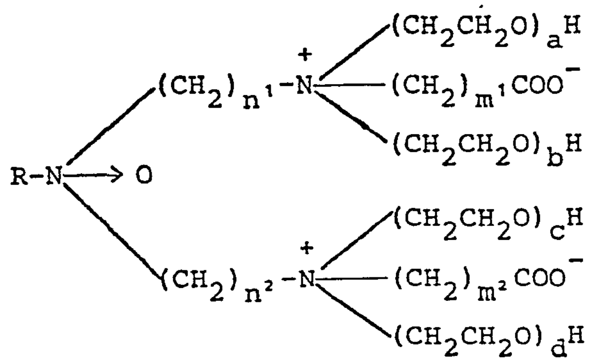

(I)

worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt,

sowie a, b, c und d, gleich oder verschieden, jeweils eine Zahl von 1 bis 5 ist, wobei die Summe a + b + c + d höchstens 10 betragen soll.

2. Verfahren zur Herstellung von Bis-Betain-Aminoxiden gemäß Anspruch 1, bei dem zunächst ein primäres Amin der Formel $RNH_2$ (II) mit 2 mol mindestens eines reaktionsfähigen Nitrils von 2 bis 3 C-Atomen zu einer Verbindung der Formel

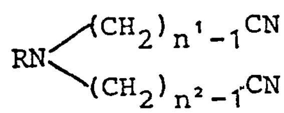

(III)

umgesetzt, in Gegenwart von Wasserstoff zu einer Verbindung der Formel

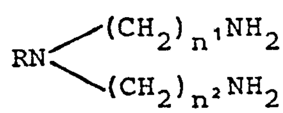

(IV)

reduziert und mit Ethylenoxid zu einer Verbindung der Formel

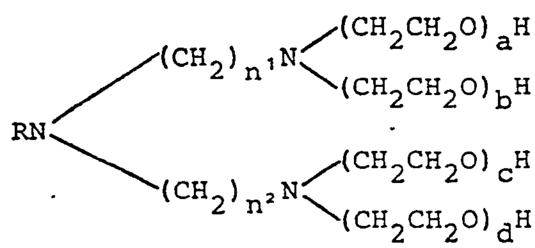

(V)

kondensiert wird, dadurch gekennzeichnet, daß diese Verbindung der Formel (V) in wäßriger Lösung mit mindestens einem Alkalisalz einer ω-Halogencarbonsäure der Formel

$$X(CH_2)_{m1\ (m^2)}COOH \quad \text{(VI)}$$

zu einem Bis-Betain der Formel

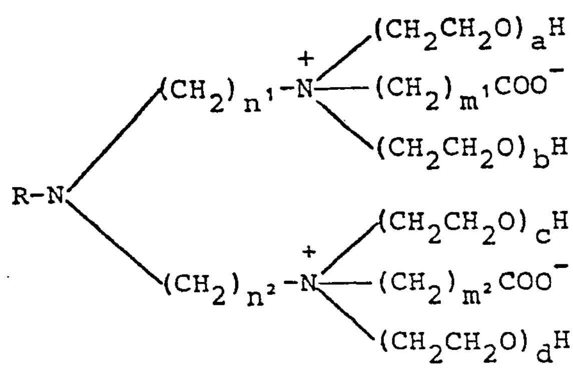

(VII)

quaterniert und das erhaltene Bis-Betain dan mit Wasserstoffperoxid zum Bis-Betain-Aminoxid oxidiert wird.

3. Kosmetisches Reinigungsmittel, enthaltend Wasser als flüssigen Träger, gegebenenfalls mindestens ein Tensid aus der Gruppe der anionischen, kationischen, nicht-ionischen oder amphoteren Tenside, gegebenenfalls übliche kosmetische Zusätze und Gerüstsubstanzen, gekennzeichnet durch einen Gehalt an einem Bis-Betain-Aminoxid der Formel (I) gemäß Anspruch 1.

4. Technisches Reinigungsmittel, enthaltend Wasser als flüssigen Träger, gegebenenfalls mindestens ein Tensid aus der Gruppe der anionischen, kationischen, nicht-ionischen oder amphoteren Tenside sowie gegebenenfalls reinigungsverstärkende Zusätze und übliche Hilfsstoffe, gekennzeichnet durch einen Gehalt an einem Bis-Betain-Aminoxid der Formel (I) gemäß Anspruch 1.

**Patentansprüche** (für den Vertragsstaat AT)

1. Verfahren zur Herstellung von Bis-Betain-Aminoxiden der allgemeinen Formel

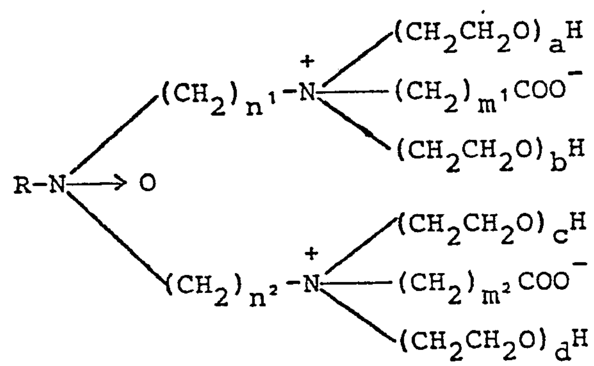

(I)

worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt,

sowie a, b, c und d, gleich oder verschieden, jeweils eine Zahl von 1 bis 5 ist, wobei die Summe a + b + c + d höchstens 10 betragen soll, bei dem zunächst ein primäres Amin der Formel $RNH_1$ (II) mit 2 mol mindestens eines reaktionsfähigen Nitrils von 2 bis 3 C-Atomen zu einer Verbindung der Formel

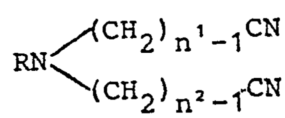

(III)

umgesetzt, in Gegenwart von Wasserstoff zu einer Verbindung der Formel

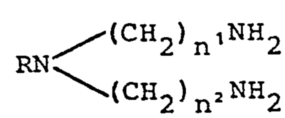

(IV)

reduziert und mit Ethylenoxid zu einer Verbindung der Formel

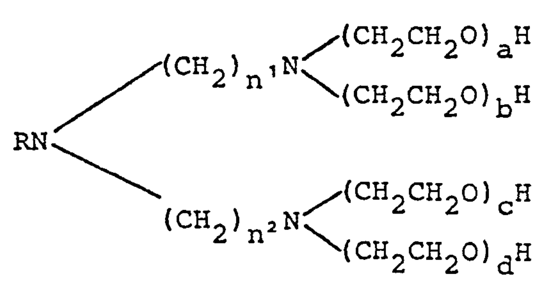

(V)

kondensiert wird, dadurch gekennzeichnet, daß diese Verbindung der Formel (V) in wäßriger Lösung mit mindestens einem Alkalisalz einer ω-Halogencarbonsäure der Formel

$$X(CH_2)_{m^1\,(m^2)}COOH \qquad (VI)$$

zu einem Bis-Betain der Formel

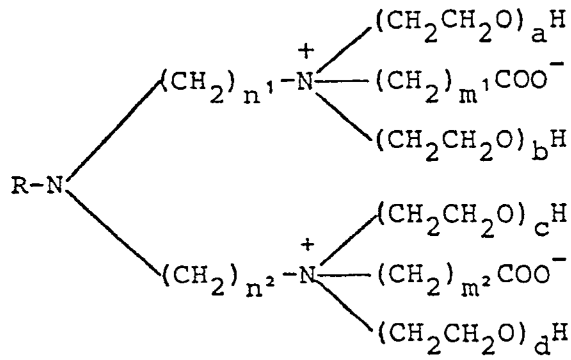

(VII)

quaterniert und das erhaltene Bis-Betain dann mit Wasserstoffperoxid zum Bis-Betain-Aminoxid oxidiert und gegebenenfalls in Substanz isoliert wird.

2. Kosmetisches Reinigungsmittel, enthaltend Wasser als flüssigen Träger, mindestens ein Tensid aus der Gruppe der anionischen, kationischen, nicht-ionischen oder amphoteren Tenside sowie gegebenenfalls übliche kosmetische Zusätze und Gerüstsubstanzen, gekennzeichnet durch einen Gehalt an einem Bis-Betain-Aminoxid der Formel (I) gemäß Anspruch 1.

3. Technisches Reinigungsmittel, enthaltend Wasser als flüssigen Träger, mindestens ein Tensid aus der Gruppe der anionischen, kationischen, nicht-ionischen oder amphoteren Tenside sowie gegebenenfalls reinigungsverstärkende Zusätze und übliche Hilfsstoffe, gekennzeichnet durch einen Gehalt an einem Bis-Betain-Aminoxid der Formel (I) gemäß Anspruch 1.

**Claims** (for the Contracting States : BE, CH, DE, FR, GB, IT, LI, NL, SE)

1. Bis-betaine-amine oxides of the formula

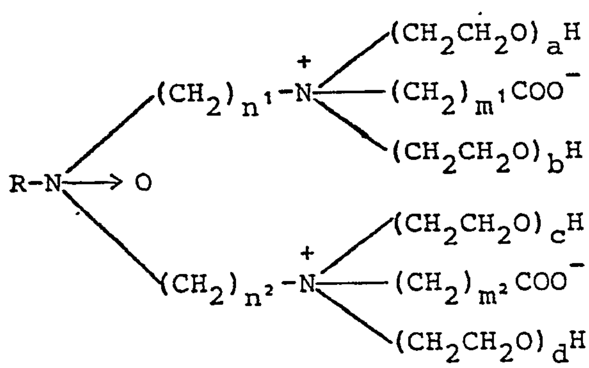

(I)

in which

R is a saturated or an olefinically unsaturated hydrocarbon radical having 1 to 3 double bonds and 8 to 22 carbons atoms,

$n^1$ and $n^2$ each is an integer of from 2 to 3, $n^1$ and $n^2$ optionally being identical or different,

$m^1$ and $m^2$ each is an integer of from 1 to 4, $m^1$ and $m^2$ optionally being identical or different,

and a, b, c and d, being identical or different, each is a number of from 1 to 5, with the proviso that the sum (a + b + c + d) is at most 10.

2. A process for the preparation of bis-betaine-amine oxides as claimed in claim 1, in which first a primary amine of the formula $RNH_2$ (II) is reacted with 2 moles of at least one reactive nitrile of 2 to 3 carbon atoms to give a compound of the formula

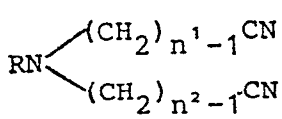

(III)

which is reduced in the presence of hydrogen to a compound of the formula

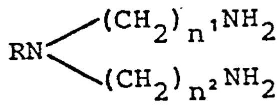

(IV)

which is condensed with ethylene oxide to give a compound of the formula

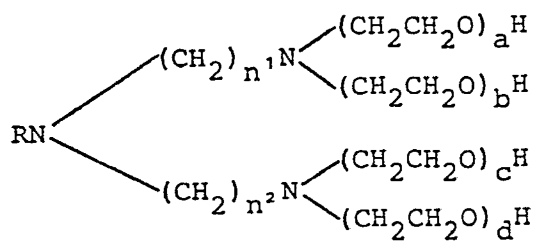

(V)

which process is characterised by quaternising the compound of the formula (V) in an aqueous solution with at least one alkali metal salt of an ω-halocarboxylic acid of the formula

$$X(CH_2)_{m^1\,(m^2)}COOH \qquad (VI)$$

to give a bis-betaine of the formula

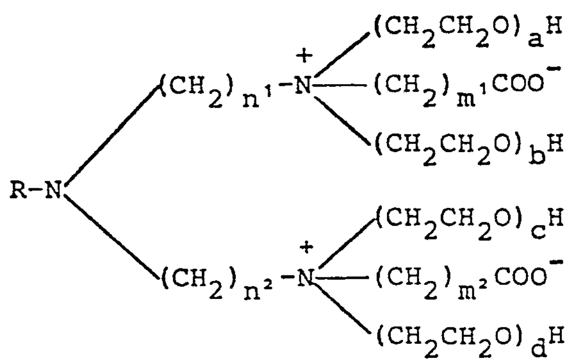

(VII)

and then oxidising the bis-betaine obtained with hydrogen peroxide to yield the bis-betaine amine oxide.

3. A cosmetic cleaning agent, containing water as liquid carrier, if appropriate, at least one surfactant from the group consisting of anionic, cationic, non-ionic and amphoteric surfactants and, if appropriate, customary cosmetic additives and auxiliaries which is characterised by an amount of a bis-betaine amine oxide as claimed in claim 1.

4. An industrial cleaning agent, containing water as liquid carrier, if appropriate, at least one surfactant from the group consisting of anionic, cationic, non-ionic, and amphoteric surfactants and, if appropriate, cleaning-promoting additives and customary auxiliaries, which is characterised by an amount of a bis-betaine-amine oxyde as claimed in claim 1.

**Claims** (for the Contracting State AT)

1. A process for the preparation of bis-betaine-amine oxides of the formula

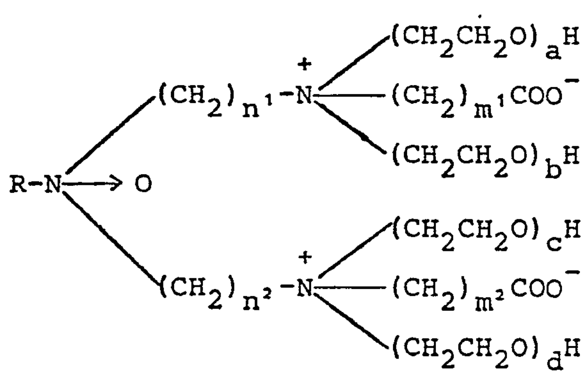

(I)

in which

R is a saturated or an olefinically unsatured hydrocarbon radical having 1 to 3 double bonds and 8 to 22 carbon atoms,

$n^1$ and $n^2$ each is an integer of from 2 to 3, $n^1$ and $n^2$ optionally being identical or different,

$m^1$ and $m^2$ each is an integer of from 1 to 4, $m^1$ and $m^2$ optionally being identical or different,

and a, b, c and d, being identical or different, each is a number of from 1 to 5, with the proviso that the sum (a + b + c + d) is at most 10, in which first a primary amine of the formula $RNH_2$ (II) is reacted with 2 moles of at least one reactive nitrile of 2 to 3 carbon atoms to give a compound of the formula

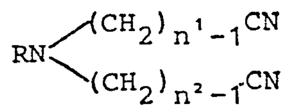

(III)

which is reduced in the presence of hydrogen to a compound of the formula

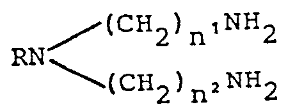

(IV)

which is condensed with ethylene oxide to give a compound of the formula

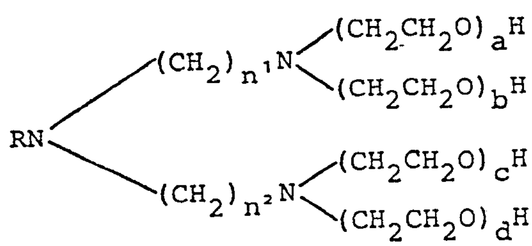

(V)

which process is characterised by quaternising the compound of the formula (V) in an aqueous solution with at least one alkali metal salt of an ω-halocarboxylic acid of the formula

$$X(CH_2)_{m^1\,(m^2)}COOH \qquad (VI)$$

to give a bis-betaine of the formula

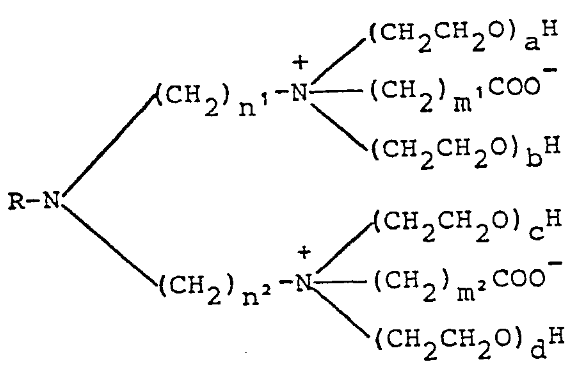

(VII)

and then oxidising the bis-betaine obtained with hydrogen peroxide to yield the bis-betaine amine oxide.

2. A cosmetic cleaning agent, containing water as liquid carrier, if appropriate, at least one surfactant from the group consisting of anionic, cationic, non ionic and amphoteric surfactants and, if appropriate, customary cosmetic additives and auxiliaries which is characterised by an amount of a bis-betaine amine oxide as claimed in claim 1.

3. An industrial cleaning agent, containing water as liquid carrier, if appropriate, at least one surfactant from the group consisting of anionic, cationic, non-ionic, and amphoteric surfactants and, if appropriate, cleaning-promoting additives and customary auxiliaries, which is characterised by an amount of a bis-betaine-amine oxide as claimed in claim 1.

**Revendications** (pour les Etats contractants : BE, CH, DE, FR, GB, IT, LI, NL, SE)

1. Bis-bétaïne-amine-oxydes de formule générale

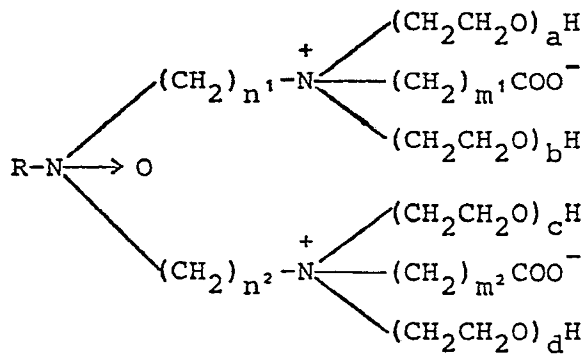

(I)

dans laquelle

R est un radical hydrocarboné saturé ou à insaturation oléfinique ayant 1 à 3 doubles liaisons et 8 à 22 atomes de carbone ;

$n^1$ et $n^2$ sont chacun un nombre entier de 2 à 3, $n^1$ et $n^2$ pouvant être identiques ou différents ;

$m^1$ et $m^2$ sont chacun un nombre entier de 1 à 4, $m^1$ et $m^2$ pouvant être identiques ou différents ;

et a, b, c et d, identiques ou différents, sont chacun un nombre de 1 à 5, la somme a + b + c + d étant au plus égale à 10.

2. Procédé pour la préparation de bis-bétaïne-amine-oxydes selon la revendication 1, dans lequel on fait d'abord réagir une amine primaire de formule $RNH_2$ (II) sur 2 moles d'au moins un nitrile réactif ayant 2 à 3 atomes de carbone, pour donner un composé de formule

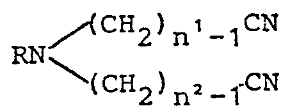

(III)

On la réduit en présence d'hydrogène pour donner un composé de formule

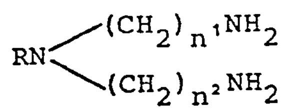

(IV)

et on la condense avec de l'oxyde d'éthylène pour donner un composé de formule

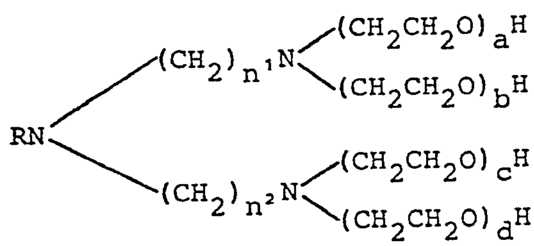

(V)

caractérisé en ce que ce composé de formule (V) est quaternarisé en solution aqueuse, avec au moins un sel alcalin d'un acide ω-halogénocarboxylique de formule

$$X(CH_2)_{m^1\,(m^2)}COOH \quad \text{(VI)}$$

pour donner une bis-bétaïne de formule

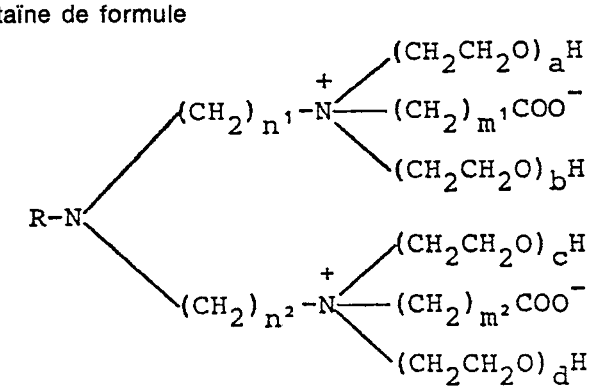

(VII)

et que l'on oxyde la bis-bétaïne obtenue avec du peroxyde d'hydrogène pour donner le bis-bétaïne-amine-oxyde.

3. Produit de nettoyage cosmétique, contenant de l'eau comme support liquide, éventuellement au moins un surfactif du groupe des surfactifs anioniques, cationiques, non ioniques ou amphotères, éventuellement des additifs cosmétiques courants et des adjuvants, caractérisé en ce qu'il contient un bis-bétaïne-amine-oxyde de formule I selon la revendication 1.

4. Produit de nettoyage technique, contenant de l'eau comme support liquide, éventuellement au moins un surfactif du groupe des surfactifs anioniques, cationiques, non ioniques ou amphotères, et éventuellement des additifs renforçateurs de nettoyage et des produits auxiliaires courants, caractérisé en ce qu'il contient un bis-bétaïne-amine-oxyde de formule I selon la revendication 1.

**Revendications** (pour l'Etat contractant AT)

1. Procédé pour la préparation de bis-bétaïne-amine-oxydes de formule générale

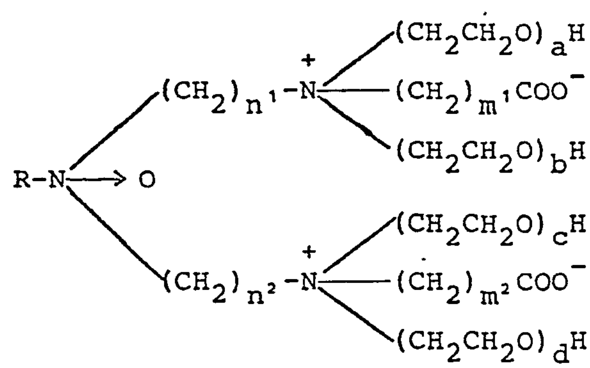

(I)

dans laquelle

R est un radical hydrocarboné saturé ou à insaturation oléfinique ayant 1 à 3 doubles liaisons et 8 à 22 atomes de carbone ;

$n^1$ et $n^2$ sont chacun un nombre entier de 2 à 3, $n^1$ et $n^2$ pouvant être identiques ou différents ;

$m^1$ et $m^2$ sont chacun un nombre entier de 1 à 4, $m^1$ et $m^2$ pouvant être identiques ou différents ;

et a, b, c et d, identiques ou différents, sont chacun un nombre de 1 à 5, la somme a + b + c + d devant être au plus égale à 10 ; dans lequel on fait d'abord réagir une amine primaire de formule $RNH_2$ (II) sur 2 moles d'au moins un nitrile réactif ayant 2 à 3 atomes de carbone pour donner un composé de formule

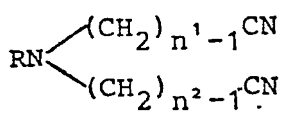

(III)

on la réduit en présence d'hydrogène pour donner un composé de formule

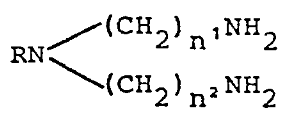

(IV)

et on la condense avec de l'oxyde d'éthylène pour donner un composé de formule

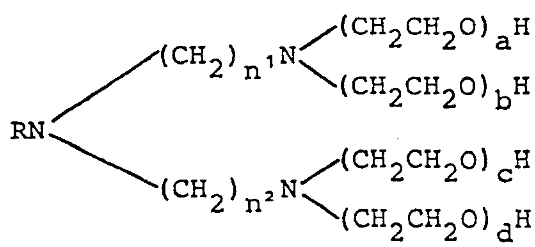

(V)

caractérisé en ce que ce composé de formule (V) est quaternarisé en solution aqueuse avec au moins un sel alcalin d'un acide ω-halogénocarboxylique de formule

$$X(CH_2)_{m^1\,(m^2)}COOH \qquad (VI)$$

pour donner une bis-bétaïne de formule

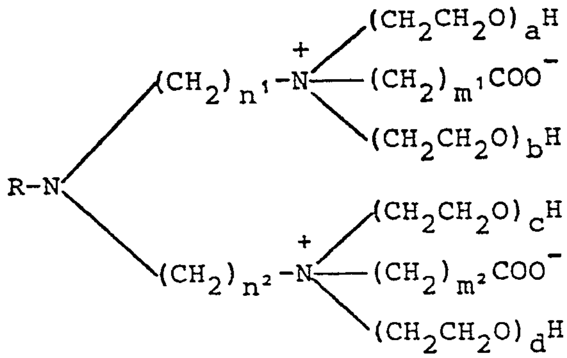

(VII)

et que la bis-bétaïne obtenue est ensuite oxydée par du peroxyde d'hydrogène pour donner la bis-bétaïne-amine-oxyde, et est éventuellement isolée telle quelle.

2. Produit de nettoyage cosmétique contenant de l'eau comme support liquide, au moins un surfactif du groupe des surfactifs anioniques, cationiques, non ioniques ou amphotères, et éventuellement des additifs cosmétiques courants et des adjuvants, caractérisé en ce qu'il contient un bis-bétaïne-amine-oxyde de formule I selon la revendication 1.

3. Produit de nettoyage technique contenant de l'eau comme support liquide, au moins un surfactif du groupe des surfactifs anioniques, cationiques, non ioniques ou amphotères, et éventuellement des additifs renforçateurs de nettoyage et des substances auxiliaires courantes, caractérisé en ce qu'il contient un bis-bétaïne-amine-oxyde de formule I selon la revendication 1.